# EUROPÄISCHE PATENTSCHRIFT

(11) **EP 0 946 861 B1**
(45) Veröffentlichungstag und Bekanntmachung des Hinweises auf die Patenterteilung: **13.03.2002**
(21) Anmeldenummer: 97946716.4
(22) Anmeldetag: 12.12.1997
(51) Int. Cl.: G01L 9/00, G01N 27/12, G01N 33/00

(54) **VERFAHREN ZUM AUFBRINGEN EINES MIKROSYSTEMS ODER WANDLERS AUF EIN SUBSTRAT UND NACH DIESEM VERFAHREN HERSTELLBARE VORRICHTUNG**
METHOD FOR APPLYING A MICROSYSTEM OR A CONVERTER ON A SUBSTRATE, AND DEVICE MANUFACTURED ACCORDINGLY
PROCEDE D'APPLICATION D'UN MICROSYSTEME OU D'UN CONVERTISSEUR SUR UN SUBSTRAT, ET DISPOSITIF FABRIQUE SELON CE PROCEDE

(30) Priorität: 17.12.1996 CH 309196
(43) Veröffentlichungstag der Anmeldung: 06.10.1999
(73) Patentinhaber: Laboratorium für Physikalische Elektronik, 8093 Zürich (CH)
(72) Erfinder: MAYER, Felix, CH-8057 Zürich (CH); PAUL, Olivier, CH-5400 Baden (CH)
(74) Vertreter: Frei, Alexandra Sarah
(86) Internationale Anmeldenummer: CH9700465
(87) Internationale Veröffentlichungsnummer: WO9827411

(56) Entgegenhaltungen:
- EP-A- 0 543 430
- EP-A- 0 654 826
- EP-A- 0 709 659
- WO-A-93/07457
- WO-A-98/05935
- US-A- 4 763 098
- PATENT ABSTRACTS OF JAPAN vol. 012, no. 317 (P-750), 29.August 1988 & JP 63 083655 A (MATSUSHITA ELECTRONICS CORP), 14.April 1988,

## Beschreibung

Die Erfindung betrifft ein Verfahren zum Aufbringen eines Mikrosystems oder Wandlers auf ein Substrat und eine nach diesem Verfahren herstellbare Vorrichtung gemäss den Oberbegriffen der unabhängigen Patentansprüche.

In dieser Schrift werden die Begriffe "Sensor", "Aktor", "Wandler" und "Mikrosystem" der Einfachheit halber unter dem Begriff "System" zusammengefasst. Bei einer Vielzahl von solchen Systemen, bspw. bei vielen Sensoren und Aktoren, müssen nebst der elektrischen Kontaktierung noch weitere Verbindungen zur Umgebung bestehen. Ein Teil des Systems, bspw. eine empfindliche Fläche eines Feuchtigkeitssensors, soll also für äussere Einflüsse, bspw, für die zu messende Aussenluft, offen sein. Mit anderen Worten: Es muss eine Verbindung zwischen einem bestimmten Teil des Systems und der Umgebung bestehen, so dass dieser Teil des Systems mit der Umgebung wechselwirken kann. Gleichzeitig sollen aber andere Teile des Systems möglichst gut vor äusseren Einflüssen, bspw. vor Feuchtigkeit, Verunreinigungen, korrosiven Dämpfen etc., geschützt sein. Solche zu schützenden Teile sind z. B. elektrische Kontakte, elektronische Funktionseinheiten, Schaltungen oder andere Sensoren, welche keiner Öffnung bedürfen, bspw. Temperatursensoren. Das System muss also lokal selektiv verpackt werden.

Bisher bekannte Herstellungsverfahren für lokal selektiv verpackte Systeme sind z. B. folgende:
- Dosiertechnik: Mit einem robotergesteuerten Dispenser wird ein Umhüllungsmaterial mit der richtigen Viskosität so auf das System aufgebracht, dass die gewünschte Fläche frei bleibt.
- Luftstromtechnik: Wiederum wird ein viskoses Umhüllungsmaterial auf das System aufgebracht. Um eine vom Umhüllungsmaterial freie Teilfläche zu erhalten, wird durch eine Düse inertes Gas derart auf die sensitive Fläche geblasen, dass dadurch die Fliessbewegung des Umhüllungsmaterials über dieser Teilfläche gestoppt wird.
- Kapillarkrafttechnik: Das zu verpackende System wird mit einem Deckblatt, welches in kleinem Abstand zur Oberfläche gehalten wird, abgedeckt. Durch Kapillarkräfte wird viskoses Umüllungsmaterial zwischen Deckblatt und System gezogen. Über der offenzuhaltenden Teilfläche ist eine Öffnung im Deckblatt angebracht, so dass dort wegen fehlender Kapillarkraft der Fluss des Umhüllungsmaterials gestoppt wird und eine Öffnung im Umhüllungsmaterial entsteht.
- UV-Licht-Technik: Es wird eine UV-härtende Vergussmasse eingesetzt. Um eine bestimmte Teilfläche des Systems offenzuhalten, wird diese Teilfläche intensiv mit UV-Licht bestrahlt. Die Vergussmasse fliesst beim Auftragen über die unbelichtete Teilfläche des Systems; am Rand der belichteten Teilfläche erstarrt sie, so dass diese Teilfläche frei bleibt.
- Stempeltechmik: Auf die offenzuhaltende Teilfläche des Systems wird ein Stempel gedrückt, während die übrige Fläche des Systems mit viskosem Umhüllungsmaterial vergossen wird.
- Spritzgusstechnik: Es werden vorgefertigte Spritzgussgehäuse verwendet, um Systeme selektiv zu verpacken.

Für die elektrische Kontaktierung des Systems wird üblicherweise die in der Mikroelektronik wohlbekannte Methode des Drahtbondens verwendet. Bei den bekannten Herstellungsverfahren für lokal selektiv verpackte Systeme werden also für die elektrische Kontaktierung und für die lokal selektive Verpackung verschiedene Prozesse, welche einander nicht unterstützen, gebraucht. Dies hat längere Herstellungszeiten, höhere Anlagen-Anschaffungskosten, einen grösseren Arbeits- und Personal aufwand und letztlich höhere Produktekosten zur Folge. Die Kosten für die Gehäusung solcher teilweise offener Systeme können denn auch einen grossen Anteil der gesamten Herstellungskosten ausmachen.

Eine Aufgabe der vorliegenden Erfindung besteht darin, ein einfaches, zuverlässiges und kostengünstiges Verfahren zum Aufbringen eines Systems auf ein Substrat anzugeben, welches gewährleistet, dass mindestens eine Teilfläche des Systems für äussere Einflüsse zugänglich und mindestens eine andere Teilfläche vor äusseren Einflüssen geschützt ist. Weiter ist es Aufgabe der vorliegenden Erfindung, eine mit diesem Verfahren einfach und kostengünstig herstellbare Vorrichtung zu schaffen.

Die Aufgabe wird gelöst durch das erfindungsgemässe Verfahren und die erfindungsgemässe Vorrichtung, wie sie in den unabhängigen Ansprüchen definiert sind.

Ein System, welches auf ein Substrat aufgebracht werden soll, weise mindestens eine erste Teilfläche, deren Wechselwirkung mit der Umgebung nach Ausführung des Verfahrens möglich sein soll, und mindestens eine zweite Teilfläche, die vor äusseren Einflüssen geschützt sein soll, auf. Im erfindungsgemässen Verfahren wird zunächst das Substrat vorbereitet, wobei mindestens eine für die vorgesehene Wechselwirkung geeignete Durchlassstelle im Substrat erzeugt wird. Eine solche Durchlassstelle ist vorzugsweise als mindestens eine Öffnung im Substrat ausgebildet. Das System und das Substrat werden derart gegenseitig positioniert, dass die mindestens eine erste Teilfläche dem Substrat zugewandt ist und dass die mindestens eine Durchlassstelle im Substrat und die mindestens eine erste Teilfläche gegeneinander zu liegen kommen. An vorgesehenen Kontaktstellen werden, vorzugsweise mit einer der bekannten Flip-Chip-Technologien, Kontakte hergestellt. Die Kontakte verbinden das System und das Substrat, und zwar mechanisch und/oder elektrisch, d. h. mindestens ein Kontakt kann elektrisch leitend sein. Ausserhalb der Kontakte weisen das System und das Substrat normalerweise einen Abstand voneinander auf, so dass mindestens ein Zwischenraum entsteht.

Mit der oben beschriebenen Grundform des erfindungsgemässen Herstellungsverfahrens ist die lokal selektive Verpackung, d. h. der Schutz der mindestens einen zweiten Teilfläche, bereits gewährleistet. Die mindestens eine zweite Teilfläche ist bspw. vor Kontakten mit äusseren Gegenständen, vor Flüssigkeitsspritzern von aussen etc. geschützt. Für viele Anwendungen wird jedoch eine hermetische Abdichtung verlangt. Eine hermetische Abdichtung kann erfindungsgemäss mit einer der folgenden drei Varianten gewährleistet werden.

In einer ersten Variante wird mindestens ein Kontakt hergestellt, welcher die mindestens eine Durchlassstelle im Substrat vollständig umgibt. Ein solcher Abdichtkontakt dichtet also die mindestens eine zweite Teilfläche hermetisch gegen äussere Einflüsse ab. Der grösste Vorteil dieser Variante besteht darin, dass für die Herstellung sowohl der elektrischen Kontaktierung als auch der lokal selektiven Verpackung derselbe Prozess verwendet wird. Dadurch wird die Herstellung besonders einfach, zuverlässig und kostengünstig.

In einer zweiten Variante wird der Zwischenraum zwischen dem System und dem Substrat unter Ausnützung von Kapillarkräften zumindest teilweise mit einem zunächst flüssigen oder zähflüssigen Füllmaterial ausgefüllt. Im Bereich der mindestens einen Durchlassstelle im Substrat sind die Kapillarkräfte zu klein oder fehlen ganz, weshalb das Füllmaterial die mindestens eine erste Teilfläche nicht benetzt. Das Füllmaterial wird anschliessend ausgehärtet und dichtet die mindestens eine zweite Teilfläche hermetisch gegen äussere Einflüsse ab, während die mindestens eine erste Teilfläche offen ist. Die Vorteile dieser Variante sind darin zu sehen, dass die Abdichtung unter Umständen wirksamer, weil vollflächig, ist. Das Füllmaterial gewährleistet gleichzeitig einen noch besseren Zusammenhalt zwischen System und Substrat. Weiter gleicht das Füllmaterial verschieden grosse thermische Ausdehnungen des Systems und des Substrates aus.

Eine dritte Variante besteht in der Kombination der ersten und zweiten Variante. Es wird also ein Abdichtkontakt um die mindestens eine erste Teilfläche hergestellt, und die mindestens eine zweite Teilfläche wird zumindest teilweise mit einem dichtenden Füllmaterial vergossen. Der Abdichtkontakt muss in dieser dritten Variante die mindestens eine Durchlassstelle nicht unbedingt vollständig umgeben; trotzdem wird die mindestens eine zweite Teilfläche hermetisch abgedichtet. Ein Vorteil dieser Variante besteht darin, dass der Abdichtkontakt als Barriere für das Füllmaterial dient und so eine exakte Abgrenzung zwischen dem vergossenen und dem nicht-vergossenen Bereich definiert. Ausserdem vereinigt diese dritte Variante die meisten Vorteile der ersten beiden Varianten.

Die mit dem obigen Verfahren herstellbare erfindungsgemässe Vorrichtung kann bspw. für Durchflussensoren, Viskositätssensoren, Feuchtigkeitssensoren, Kraftsensoren, Drucksensoren, Sensoren für elektromagnetische Strahlung, Sensoren für Teilchenstrahlung oder chemische Sensoren verwendet werden.

Im folgenden wird die Erfindung anhand der Figuren detailliert erläutert. Dabei zeigen schematisch:
- Fig. 1-5: verschiedene Schritte einer ersten Ausführungsform des erfindungsgemässen Herstellungsverfahrens im Querschnitt,
- Fig. 6: eine Draufsicht auf die erste Ausführungsform der erfindungsgemässen Vorrichtung,
- Fig. 7: einen Querschnitt durch eine zweite Ausführungsform der erfindungsgemässen Vorrichtung,
- Fig. 8 und 9: eine Draufsicht auf bzw. einen Querschnitt durch eine dritte Ausführungsform der erfindungsgemässen Vorrichtung und
- Fig. 10: eine dreidimensionale Darstellung eines mit dem erfindungsgemässen Verfahren hergestellten Flussensors.

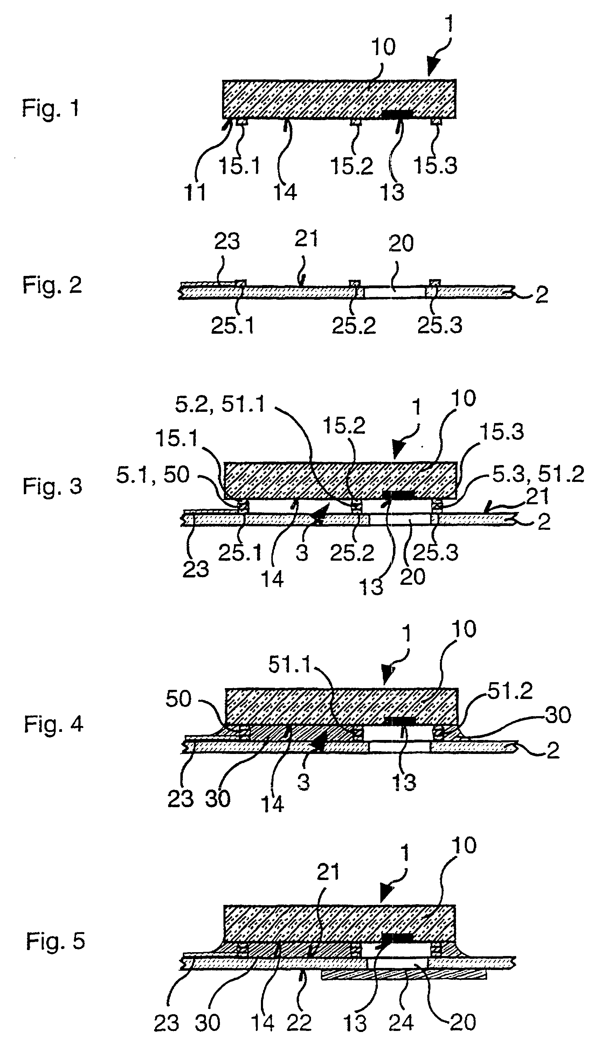

Um das Verständnis zu erleichtern, sind in den Figuren 1-5, 7, 9 und 10 die Höhen gegenüber den Längen nicht massstäblich gezeichnet; auch stehen die Höhen der einzelnen Elemente nicht unbedingt im richtigen Verhältnis zueinander.

Die Figuren 1-5 zeigen schematisch verschiedene Schritte einer Ausführungsform des erfindungsgemässen Herstellungsverfahrens. Die Verfahrensschritte können in der hier beschriebenen oder auch in anderer Reihenfolge ausgeführt werden. Es sind jeweils Querschnitte durch Zwischenprodukte bzw. durch ein fertiges Produkt nach den jeweiligen Verfahrensschritten dargestellt.

**Figur 1** zeigt schematisch ein System 1, welches zum Verpacken vorbereitet ist. Es beinhaltet einen Träger 10, vorzugsweise aus einem Halbleitermaterial wie Silizium. Auf mindestens einer Seite 11 des Systems 1 befinden sich typischerweise elektronische, mechanische, thermische, chemische und/oder andere Funktionseinheiten, welche jedoch der Übersichtlichkeit halber nicht eingezeichnet sind. Diese Funktionseinheiten bestimmen im wesentlichen die Funktion des Systems 1. Sie werden mit aus der Mikroelektronik und Mikrosystemtechnik bekannten Verfahren wie Epitaxie, Oxidation, Photolithographie, Diffusion, Ionenimplantation, Metallisierung, anisotropes Ätzen, oder auch mit anderen, auf eine jeweilige Verwendung speziell zugeschnittenen Verfahren hergestellt.

Auf einer Seite 11 des Systems 1 befindet sich eine erste Teilfläche 13, welche in der Anwendung für äussere Einflüsse zugänglich sein soll. Wenn es sich beim System 1 bspw. um einen Sensor handelt, so enthält die erste Teilfläche 13 die sensitive Fläche des Sensors, auf welche von aussen ein zu messendes Signal einwirkt. Obwohl in Fig. 1 nur eine einzige erste Teilfläche 13 eingezeichnet ist, kann das System 1 auch mit mehreren ersten Teilflächen versehen sein. Eine zweite Teilfläche 14 des Systems 1 soll vor äusseren Einflüssen geschützt werden. Bei einem Mikrosystem 1 mit einem Sensor befindet sich auf der zweiten Teilfläche 14 bspw. eine Auswertelektronik, welche weder durch das zu messende Signal noch durch andere äussere Einflüsse beeinflusst oder beschädigt werden darf. Auf derselben Seite 11 wie die mindestens eine erste Teilfläche 13 und zweite Teilfläche 14 befinden sich vorbereitete Kontaktstellen 15.1-15.3, auf welche aber, je nach Kontaktierungsmethode, auch verzichtet werden kann.

**Figur 2** zeigt schematisch ein Substrat 2, auf welches das System 1 aufgebracht werden soll. Das Substrat 2 besteht bspw. aus Keramik, Kunststoff wie z. B. Epoxidharz, FR4, Polyimid oder einem anderen Kunststoff und ist vorzugsweise als Leiterplatte (printed circuit board, PCB) ausgebildet. Es kann bspw. nach dem DYCOstrate®-Verfahren, wie in den Schriften WO92/15408 und WO93/26143 offenbart, hergestellt sein. Das Substrat 2 ist mit mindestens einer Durchlassstelle 20 ausgestattet, welche der mindestens einen ersten Teilfläche 13 auf dem System 1 entspricht. Die mindestens eine Durchlassstelle 20 ist derart beschaffen, dass sie für die vorgesehene Wechselwirkung der mindestens einen ersten Teilfläche 13 mit der Umgebung geeignet ist. Sie kann bswp. als eine oder mehrere Öffnungen im Substrat 2, als fein perforierte Substratstelle, als Substratstelle mit anderen Materialeigenschaften, als besonders dünne Substratstelle etc. ausgebildet sein. In den folgenden Figuren ist die Durchlassstelle 20 jeweils der Einfachheit halber als Öffnung 20 dargestellt bzw. beschrieben. Wie das System 1, so kann fakultativ auch das Substrat 2 auf einer Seite 21 vorbereitete Kontaktstellen 25.1-25.3 aufweisen. Das Substrat 2 kann mit elektrischen Leiterbahnen 23 ausgestattet sein, die bspw. in PCB-Technologie oder in Dickfilmtechnologie hergestellt wurden.

Gemäss dem erfindungsgemässen Verfahren werden, wie in **Figur 3** dargestellt, das System 1 und das Substrat 2 derart gegenseitig positioniert, dass die erste Teilfläche 13 dem Substrat 2, d. h. seiner eventuell mit Kontaktstellen 25.1-25.3 versehenen Seite 21, zugewandt ist und dass die Öffnung 20 im Substrat 2 und die erste Teilfläche 13 gegeneinander zu liegen kommen, dass also die Öffnung 20 die erste Teilfläche 13 frei lässt. An den vorgesehenen, eventuell vorbereiteten Kontaktstellen 15.1-15.3, 25.1-25.3 werden Kontakte 5.1-5.3 hergestellt, bspw. durch Verlöten oder durch Kleben. Die Lötpaste oder der Kleber können bspw. mittels Schablonendruck, Siebdruck, Tauchen in schmelzflüssiges Lot oder galvanisch auf die Kontaktstellen 15.1-15.3, 25.1-25.3 aufgebracht werden. Solche Methoden zum Aufbringen und Verlöten eines Halbleiters 1 auf ein Substrat 2 sind bekannt und werden als "Flip-Chip-Technologien" bezeichnet.

Die Kontakte 5.1-5.3 können unter anderem dazu dienen, das System 1 und das Substrat 2 zusammenzuhalten. Es ist aber vorteilhaft, die Kontakte 5.1-5.3 gleichzeitig für weitere Funktionen zu verwenden. Gewisse Kontakte 50 können für eine elektrische Funktion vorgesehen sein, gewisse Kontakte 51.1, 51.2 zum Abdichten der zweiten Teilfläche 14. Ein Kontakt kann auch mehrere Funktionen ausüben. Falls der Kontakt 51.1, 51.2 bei der Öffnung 20 die Öffnung 20 vollständig umgibt, bildet er einen Abdichtkontakt, und die in Fig. 3 dargestellte Ausführungsform der erfindungsgemässen Vorrichtung entspricht der ersten obenerwähnten Variante einer lokal selektiven Verpackung mit hermetischer Abdichtung. Ausserhalb der Kontakte 50, 51.1, 51.2 weisen das System 1 und das Substrat 2 normalerweise einen Abstand voneinander auf, so dass mindestens ein Zwischenraum 3 entsteht.

**Figur 4** zeigt eine Ausführungsform der erfindungsgemässen Vorrichtung nach einem weiteren, fakultativen Verfahrensschritt. In diesem Verfahrensschritt wird der Zwischenraum 3 zwischen dem System 1 und dem Substrat 2 unter Ausnützung von Kapiliarkräften zumindest teilweise mit einem zunächst flüssigen oder zähflüssigen Füllmaterial 30 ausgefüllt. Der Abdichtkontakt 51.1, 51.2 verhindert in dieser Ausführungsform, dass das Füllmaterial 30 zur ersten Teilfläche 13 gelangt. Das Füllmaterial 30 wird anschliessend ausgehärtet und dichtet die zweite Teilfläche 14 hermetisch gegen äussere Einflüsse ab, während die erste Teilfläche 13 offen bleibt. Eine vorteilhafte Nebenwirkung des Füllmaterials 30 besteht darin, dass es die verschieden grossen Ausdehnungen des Systems 1 und des Substrates 2 ausgleicht. Der Träger 10 des Systems 1 und das Substrat 2 weisen nämlich im Normalfall verschiedene thermische Ausdehnungskoeffizienten auf, was sich nachteilig auf die Langzeitstabilität auswirken kann. Die in Fig. 4 dargestellte Ausführungsform der erfindungsgemässen Vorrichtung entspricht der dritten obenerwähnten Variante einer lokal selektiven Verpackung mit hermetischer Abdichtung.

**Figur 5** zeigt eine Ausführungsform der erfindungsgemässen Vorrichtung nach einem weiteren, fakultativen Verfahrensschritt. In diesem Schritt wird die Öffnung 20 mit einer selektiven Abdeckung 24 abgedeckt. Die Abdeckung 24 ist selektiv in dem Sinne, dass sie den erwünschten Kontakt zur Umgebung ermöglicht, bspw. das vom Sensor zu messende Signal auf die sensitive Sensorfläche 13 durchlässt, aber unerwünschte äussere Einflüsse blockiert. Im Falle von Infrarotsensoren könnte die Abdeckung 24 bspw. ein als Siliziumplättchen ausgebildeter Infrarot-Transmissionsfilter sein, welcher auf die Öffnung 20 aufgebracht wird. Im Falle von Feuchtigkeitssensoren könnte die Abdeckung 24 bspw. eine teildurchlässige Membran oder ein teildurchlässiges Metall (z. B. poröses Aluminium) sein. Die Abdeckung 24 kann auf der dem System 1 zugewandten Seite 21 oder der vom System 1 abgewandten Seite 22 über der Öffnung 20 oder auch in der Öffnung 20 selbst angebracht sein.

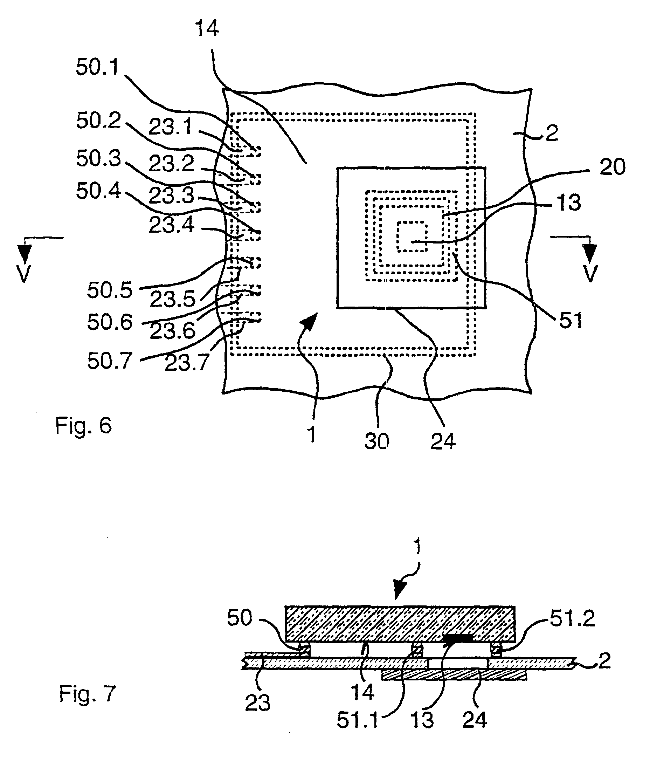

In **Figur 6** ist eine Draufsicht auf eine erste Ausführungsform einer erfindungsgemässen Vorrichtung dargestellt. Die mit V-V bezeichnete Linie deutet an, wo die in den Figuren 5 und 7 dargestellten Querschnitte gelegt wurden. Ein Substrat 2 ist mit einer Öffnung 20 versehen, welche eine Wechselwirkung einer ersten Teilfläche 13 eines Systems 1 mit der Umgebung gewährleistet. Die Öffnung 20 ist in diesem Beispiel quadratisch, kann aber auch eine andere Form haben, bspw. rechteckig oder rund. In dieser Ausführungsform umgibt ein Abdichtkontakt 51 zwischen dem System 1 und dem Substrat 2 die Öffnung bzw. die erste Teilfläche 13 vollständig und schützt, zusammen mit dem Substrat 2, eine zweite Teilfläche 14 vor äusseren Einflüssen. Der Abdichtkontakt 51 bildet eine Art Ring, welcher jedoch nicht kreisförmig zu sein braucht, um die Öffnung 20 herum. Insbesondere dann, wenn keine hermetische Abdichtung verlangt wird, muss aber der Kontakt 51 bei der Öffnung 20 die Öffnung 20 nicht vollständig umgeben. Elektrische Kontakte 50.1-50.7 können mit demselben Prozess hergestellt werden wie der Abdichtkontakt 51. Auf dem Substrat 2 sind Leiterbahnen 23.1-23.7 angebracht.

Die zweite Teilfläche 14 in Fig. 6 kann mit einem Füllmaterial 30, das in Fig. 4 und 5 gut sichtbar ist, vergossen sein. Wenn aber ein Abdichtkontakt 51 vorhanden ist, so kann auf das Füllmaterial 30 verzichtet werden, denn der Abdichtkontakt 51 verhindert schon, zusammen mit dem Substrat 2, dass die zweite Teilfläche 14 für äussere Einflüsse zugänglich ist. Eine solche zweite Ausführungsform der erfindungsgemässen Vorrichtung ohne Füllmaterial zeigt im Querschnitt **Figur 7**. Die hier dargestellte Ausführungsform der erfindungsgemässen Vorrichtung entspricht der ersten obenerwähnten Variante einer lokal selektiven Verpackung mit hermetischer Abdichtung.

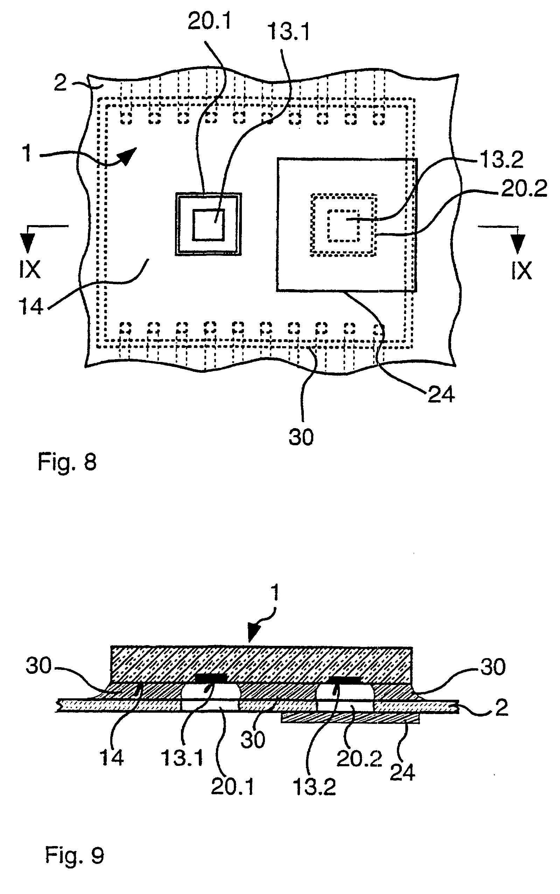

**Figur 8** zeigt eine dritte Ausführungsform der erfindungsgemässen Vorrichtung. Diese Ausführungsform hat keinen Abdichtkontakt, entspricht also der zweiten obenerwähnten Variante einer lokal selektiven Verpackung mit hermetischer Abdichtung. Der Schutz der zweiten Teilfläche 14 wird durch Füllmaterial 30 zwischen dem System 1 und dem Substrat 2 gewährleistet, wogegen die ersten Teilflächen 13.1, 13.2 unter Öffnungen 20.1, 20.2 frei sind. Die in Fig. 8 dargestellte Ausführungsform weist zwei Öffnungen 20.1, 20,2 auf; eine Öffnung 20.2 ist mit einer selektiven Abdekung 24 versehen, die andere Öffnung 20.1 nicht.

Ein Querschnitt entlang der Linie IX-IX in Fig. 8 ist in **Figur 9** dargestellt. Beim Vergiessen ist unter Ausnützung von Kapillarkräften der Zwischenraum zwischen dem System 1 und dem Substrat 2 mit flüssigem oder zähflüssigem Füllmaterial 30 ausgefüllt worden, welches anschliessend ausgehärtet wurde. Im Bereich der Öffnungen 20.1, 20.2 waren die Kapillarkräfte aber zu klein oder fehlten ganz, so dass die ersten Teilflächen 13.1, 13.2 nicht mit Füllmaterial 30 benetzt wurden. So kann auch ohne Abdichtkontakt auf einfache Weise erreicht werden, dass die ersten Teilflächen 13.1, 13.2 für äussere Einflüsse zugänglich sind, während die zweite Teilfläche 14 vor äusseren Einflüssen hermetisch abgedichtet und also geschützt ist.

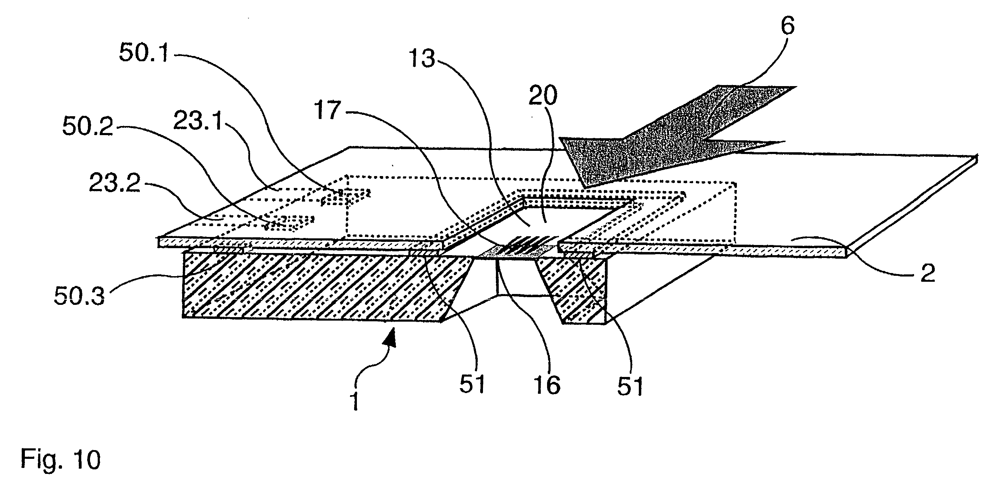

**Figur 10** zeigt schliesslich eine perspektivische, teilweise offengelegte Ansicht einer erfindungsgemässen Vorrichtung, welche als Gasflussensor verwendet wird. In dieser Darstellung befindet sich das als Sensor-Siliziumchip ausgebildete System 1 unten und das Keramiksubstrat 2 oben. Ein zu messendes Gas strömt über das Substrat, angedeutet mit einem Pfeil 6. Die Öffnung 20 im Substrat 2 lässt das Gas 6 mit einer ersten Teilfläche 13 des Sensorchips 1 in Wechselwirkung treten. Die erste Teilfläche 13 enthält eine Membran 16 aus dielektrischen Schichten. Auf der ersten Teilfläche 13 befindet sich eine integrierte Anordnung 17 von Heizwiderständen und Thermosäulen; Signale der Thermosäulen ergeben ein Mass für den Gasfluss 6.

Bei der Herstellung der Vorrichtung von Fig. 10 werden Kontaktstellen aus Gold mit einem aus der Herstellung von integrierten Schaltkreisen (IC) bekannten Standardprozess auf dem Sensorchip 1 vorbereitet. Die elektrischen Leiterbahnen 23.1, 23.2 auf dem Keramiksubstrat 2 werden mit Dickfilmtechnologie hergestellt. Auf die vorbereiteten Kontaktstellen wird mittels Schablonendruck Lötpaste aufgetragen, und beim Zusammenfügen von Sensorchip und Keramiksubstrat werden an den Kontaktstellen Kontakte 50.1-50.3, 51 durch Löten hergestellt. Ein Kontakt 51 umgibt vollständig die Öffnung 20 bzw. die erste Teilfläche 13 und dient als Abdichtkontakt. Weitere Kontakte 50.1-50.3 dienen der elektrischen Kontaktierung zwischen Sensorchip 1 und Keramiksubstrat 2.

Das erfindungsgemässe Verfahren und die erfindungsgemässe Vorrichtung können nicht nur für Gasflussensoren, sondern z. B. auch für andere Durchflussensoren, Feuchtigkeitssensoren, Infrarot-, Ultraviolett- und Lichtsensoren, chemische Sensoren oder Drucksensoren verwendet werden.

## Patentansprüche

1. Verfahren zum Aufbringen eines Mikrosystems oder Wandlers (1) auf ein Substrat (2), wobei das Mikrosystem oder der Wandler (1) mindestens eine erste Teilfläche (13), deren Wechselwirkung mit der Umgebung nach Ausführung des Verfahrens möglich sein soll, aufweist, **gekennzeichnet durch** folgende Verfahrensschritte:
a) Vorbereitung des Mikrosystems oder Wandlers (1), wobei mindestens eine zweite Teilfläche (14) mit elektronischen Funktionseinheiten versehen wird;
b) Vorbereitung eines Substrates (2), wobei mindestens eine für die Wechselwirkung geeignete Durchlassstelle (20) im Substrat (2) erzeugt wird;
c) gegenseitiges Positionieren des Mikrosystems bzw. Wandlers (1) und des Substrates (2), derart, dass die mindestens eine erste Teilfläche (13) und die mindestens eine zweite Teilfläche (14) dem Substrat (2) zugewandt sind und dass die mindestens eine Durchlassstelle (20) im Substrat (2) und die mindestens eine erste Teilfläche (13) gegeneinander zu liegen kommen;
d) Herstellung von Kontakten (5.1-5.3, 50, 51), welche das Mikrosystem bzw. den Wandler (1) und das Substrat (2) verbinden, zwischen dem Mikrosystem bzw. Wandler (1) und dem Substrat (2).

2. Verfahren nach Anspruch 1, **dadurch gekennzeichnet, dass** die Kontakte (5.1-5.3, 50, 51) zwischen dem Mikrosystem bzw. Wandler (1) und dem Substrat (2) mit einer Flip-Chip-Technologie hergestellt werden.

3. Verfahren nach Anspruch 1 oder 2, **dadurch gekennzeichnet, dass** zur hermetischen Abdichtung mindestens einer zweiten Teilfläche (14) des Mikrosystems oder Wandlers (1), die vor äusseren Einflüssen geschützt sein soll, mindestens ein Kontakt (51) zwischen dem Mikrosystem bzw. Wandler (1) und dem Substrat (2) hergestellt wird, welcher die mindestens eine Durchlassstelle (20) im Substrat (2) vollständig umgibt.

4. Verfahren nach einem der Ansprüche 1-3, **dadurch gekennzeichnet, dass** zur hermetischen Abdichtung mindestens einer zweiten Teilfläche (14) des Mikrosystems oder Wandlers (1), die vor äusseren Einflüssen geschützt sein soll, ein Zwischenraum (3) zwischen dem Mikrosystem bzw. Wandler (1) und dem Substrat (2) zumindest teilweise mit einem zunächst flüssigen oder zähflüssigen Füllmaterial (30) ausgefüllt wird.

5. Verfahren nach einem der Ansprüche 1-3 und Anspruch 4, **dadurch gekennzeichnet, dass** zur hermetischen Abdichtung mindestens einer zweiten Teilfläche (14) des Mikrosysterns oder Wandlers (1), die vor äusseren Einflüssen geschützt sein soll, mindestens ein Kontakt (51) zwischen dem Mikrosystem bzw. Wandler (1) und dem Substrat (2) hergestellt wird, welcher die mindestens eine Durchlassstelle (20) im Substrat (2) vollständig oder teilweise umgibt, und ein Zwischenraum (3) zwischen dem Mikrosystem bzw. Wandler (1) und dem Substrat (2) zumindest teilweise mit einem zunächst flüssigen oder zähflüssigen Füllmaterial (30) ausgefüllt wird.

6. Verfahren nach Anspruch 4 oder 5, **dadurch gekennzeichnet, dass** zum Ausfüllen des Zwischenraums (3) zwischen dem Mikrosystem bzw. Wandler (1) und dem Substrat (2) Kapillarkräfte ausgenützt werden.

7. Verfahren nach einem der Ansprüche 4-6, **dadurch gekennzeichnet, dass** das Füllmaterial (30) während des Vergiessens oder nach dem Vergiessen ausgehärtet wird.

8. Verfahren nach einem der Ansprüche 1-7, **dadurch gekennzeichnet, dass** als Durchlassstelle (20) mindestens eine Öffnung im Substrat (2) erzeugt wird.

9. Verfahren nach Anspruch 8, **dadurch gekennzeichnet, dass** zum Abhalten von unerwünschten äusseren Einflüssen von der ersten Teilfläche (13) die mindestens eine Öffnung (20) mit einer selektiven Abdeckung (24) abgedeckt wind.

10. Vorrichtung, herstellbar mit dem Verfahren nach einem der Ansprüche 1-9, mit einem auf einem Substrat (2) aufgebrachten Mikrosystem oder Wandler (1), wobei
sich mindestens ein Kontakt (5.1-5.3, 50, 51) zwischen dem Mikrosystem bzw. Wandler (1) und dem Substrat (2) befindet,
das Mikrosystem oder der Wandler (1) mindestens eine erste Teilfläche (13) beinhaltet, welche dem Substrat (2) zugewandt ist und mindestens einer für eine Wechselwirkung der mindestens einen ersten Teilfläche (13) mit der Umgebung geeigneten Durchlassstelle (20) im Substrat (2) gegenüberliegt,
das Mikrosystem oder der Wandler (1) mindestens eine zweite Teilfläche (14) beinhaltet, welche dem Substrat (2) zugewandt ist und zumindest einem Teil des Substrates (2) gegenüberliegt,
**dadurch gekennzeichnet,**
**dass** die mindestens eine zweite Teilfläche (14) elektronische Funktionseinheiten aufweist.

11. Vorrichtung nach Anspruch 10, **dadurch gekennzeichnet, dass** sich zwischen dem Mikrosystem bzw. Wandler (1) und dem Substrat (2) mindestens ein elektrisch leitender Kontakt (50) befindet.

12. Vorrichtung nach Anspruch 10 oder 11, **dadurch gekennzeichnet, dass** sich zwischen dem Mikrosystem bzw. Wandler (1) und dem Substrat (2) mindestens ein Abdichtkontakt (51) befindet, welcher die mindestens eine Durchlassstelle (20) im Substrat (2) vollständig umgibt.

13. Vorrichtung nach einem der Ansprüche 10-12, **dadurch gekennzeichnet, dass** sich ein Zwischenraum (3) zwischen dem Mikrosystem bzw. Wandler (1) und dem Substrat (2) befindet, welcher zumindest teilweise mit einem Füllmaterial (30) ausgefüllt ist.

14. Vorrichtung nach den Ansprüchen 12 und 13, **dadurch gekennzeichnet, dass** sich zwischen dem Mikrosystem bzw. Wandler (1) und dem Substrat (2) mindestens ein Kontakt (50) befindet, welcher die mindestens eine Durchlassstelle (20) im Substrat (2) vollständig oder teilweise umgibt, und dass sich ein Zwischenraum (3) zwischen dem Mikrosystem bzw. Wandler (1) und dem Substrat (2) befindet, welcher zumindest teilweise mit einem Füllmaterial (30) ausgefüllt ist.

15. Vorrichtung nach einem der Ansprüche 10-14, **dadurch gekennzeichnet, dass** die Durchlassstelle (20) als mindestens eine Öffnung im Substrat ausgebildet ist.

16. Vorrichtung nach Anspruch 15, **gekennzeichnet durch** mindestens eine selektive Abdeckung (24), mittels welcher unerwünschte äussere Einflüsse von der ersten Teilfläche (13) abhaltbar sind, wobei sich die mindestens eine selektive Abdeckung auf, in und/oder unter der mindestens einen Öffnung (20) befindet.

17. Vorrichtung nach Anspruch 16, **dadurch gekennzeichnet, dass** die selektive Abdeckung (24) aus porösem Metall oder aus einer teildurchlässigen Membran besteht.

18. Vorrichtung nach einem der Ansprüche 10-17, **dadurch gekennzeichnet, dass** das Mikrosystem oder der Wandler (1) als Halbleiterchip ausgebildet ist.

19. Vorrichtung nach einem der Ansprüche 10-18, **dadurch gekennzeichnet, dass** das Substrat (2) aus Keramik, aus Epoxidharz, FR4, Polyimid oder einem anderen Kunststoff besteht.

20. Vorrichtung nach einem der Ansprüche 10-19, **dadurch gekennzeichnet, dass** die Kontakte (50, 51) aus Lötmaterial oder aus Klebstoff bestehen.

21. Verwendung der Vorrichtung nach einem der Ansprüche 10-20 für Durchflussensoren, Viskositätssensoren, Feuchtigkeitssensoren, Kraftsensoren, Drucksensoren, Sensoren für elektromagnetische Strahlung, Sensoren für Teilchenstrahlung oder chemische Sensoren.

## Claims

1. Method for fitting a microsystem or converter (1) to a substrate (2), with the microsystem or the converter (1) having at least one first surface element (13) whose interaction with the environment is intended to be possible by carrying out the method, **characterized by** the following method steps:
a) Preparation of the microsystem or converter (1), with at least one second surface element (14) being provided with electronic functional units;
b) Preparation of a substrate (2), with at least one aperture point (20), which is suitable for the interaction, being produced in the substrate (2);
c) Mutual positioning of the microsystem or converter (1) and of the substrate (2) in such a manner that the at least one first surface element (13) and the at least one second surface element (14) faces the substrate (2), and in that the at least one aperture point (20) in the substrate (2) and the at least one first surface element (13) are located opposite one another;
d) Production of contacts (5.1-5.3, 50, 51), which connect the microsystem or the converter (1) and the substrate (2), between the microsystem or converter (1) and the substrate (2).

2. Method according to Claim 1, **characterized in that** the contacts (5.1-5.3, 50, 51) between the microsystem or converter (1) and the substrate (2) are produced using flipchip technology.

3. Method according to Claim 1 or 2, **characterized in that**, for hermetic sealing of at least one second surface element (14) of the microsystem or converter (1), which is intended to be protected against external influences, at least one contact (51) is produced between the microsystem or converter (1) and the substrate (2), and completely surrounds the at least one aperture point (20) in the substrate (2).

4. Method according to one of Claims 1-3, **characterized in that**, for hermetic sealing of at least one second surface element (14) of the microsystem or converter (1), which is intended to be protected against external influences, an intermediate space (3) between the microsystem or converter (1) and the substrate (2) is at least partially filled with an initially liquid or viscous filling material (30).

5. Method according to one of Claims 1-3 and Claim 4, **characterized in that**, for hermetic sealing of at least one second surface element (14) of the microsystem or converter (1), which is intended to be protected against external influences, at least one contact (51) is produced between the microsystem or converter (1) and the substrate (2) and completely or partially surrounds the at least one aperture point (20) in the substrate (2), and an intermediate space (3) between the microsystem or converter (1) and the substrate (2) is at least partially filled with an initially liquid or viscous filling material (30).

6. Method according to Claim 4 or 5, **characterized in that** capillary forces are utilized in order to fill the intermediate space (3) between the microsystem or converter (1) and the substrate (2).

7. Method according to one of Claims 4-6, **characterized in that** the filling material (30) is cured during the casting process or after the casting process.

8. Method according to one of Claims 1-7, **characterized in that** at least one opening is produced in the substrate (2) as the aperture point (20).

9. Method according to Claim 8, **characterized in that**, in order to keep undesirable external influences away from the first surface element (13), the at least one opening (20) is covered by a selective covering (24).

10. Apparatus which can be produced using the method according to one of Claims 1-9, having a microsystem or converter (1) which is fitted on a substrate (2), with
at least one contact (5.1-5.3, 50, 51) being located between the microsystem or converter (1) and the substrate (2),
the microsystem or the converter (1) containing at least one first surface element (13) which faces the substrate (2) and is opposite at least one aperture point (20) in the substrate (2), which is suitable for interaction of the at least one first surface element (13) with the environment,
the microsystem or the converter (1) containing at least one second surface element (14) which faces the substrate (2) and is opposite at least a portion of the substrate (2),
**characterized**
**in that** the at least one second surface element (14) has electronic functional units.

11. Apparatus according to Claim 10, **characterized in that** at least one electrically conductive contact (50) is located between the microsystem or converter (1) and the substrate (2).

12. Apparatus according to Claim 10 or 11, **characterized in that** at least one sealing contact (51) is located between the microsystem or converter (1) and the substrate 2 and completely surrounds the at least one aperture point (20) in the substrate (2).

13. Apparatus according to one of Claims 10-12, **characterized in that** an intermediate space (3) is located between the microsystem or converter (1) and the substrate (2) and is at least partially filled with a filling material (30).

14. Apparatus according to Claims 12 and 13, **characterized in that** at least one contact (50) is located between the microsystem or converter (1) and the substrate (2) and completely or partially surrounds the at least one aperture point (20) in the substrate (2), and **in that** an intermediate space (3) is located between the microsystem or converter (1) and the substrate (2) and is at least partially filled with a filling material (30).

15. Apparatus according to one of Claims 10-14, **characterized in that** the aperture point (20) is in the form of at least one opening in the substrate.

16. Apparatus according to Claim 15, **characterized by** at least one selective covering (24), by means of which undesirable external influences can be kept away from the first surface element (13), with the at least one selective covering being located on, in and/or under the at least one opening (20).

17. Apparatus according to Claim 16, **characterized in that** the selective covering (24) is composed of porous metal or of a semipermeable membrane.

18. Apparatus according to one of Claims 10-17, **characterized in that** the microsystem or the converter (1) is in the form of a semiconductor chip.

19. Apparatus according to one of Claims 10-18, **characterized in that** the substrate (2) is composed of ceramic, epoxy resin, FR4, polyimide or some other plastic.

20. Apparatus according to one of Claims 10-19, **characterized in that** the contacts (50, 51) are composed of soldered material or of adhesive.

21. Use of the apparatus according to one of Claims 10-20 for flow sensors, viscosity sensors, humidity or moisture sensors, force sensors, pressure sensors, sensors for electromagnetic radiation, sensors for particle radiation, or chemical sensors.

## Revendications

1. Procédé pour l'application d'un microsystème ou convertisseur (1) sur un substrat (2), le microsystème ou le convertisseur (1) présentant au moins une première surface partielle (13) dont les interactions avec l'environnement doivent être possibles après l'exécution du procédé, **caractérisé par** les étapes de procédé suivantes :
a) préparation du microsystème ou convertisseur (1), une deuxième surface partielle (14) étant dotée d'entités fonctionnelles électroniques ;
b) préparation du substrat (2), au moins un emplacement de passage (20) convenant pour l'interaction étant créé dans le substrat (2) ;
c) positionnement mutuel du microsystème ou convertisseur (1) et du substrat (2) de telle sorte que la première surface partielle (13) au moins présente et la deuxième surface partielle (14) au moins présente soient tournées vers le substrat (2) et que l'emplacement de passage (20) au moins présent dans le substrat (2) et la première surface partielle (13) au moins présente viennent de se placer l'un contre l'autre ;
d) réalisation de contacts (5.1-5.3, 50, 51) qui relient le microsystème ou le convertisseur (1) et le substrat (2) entre le microsystème ou convertisseur (1) et le substrat (2).

2. Procédé selon la revendication 1, **caractérisé en ce que** les contacts (5.1-5.3, 50, 51) entre le microsystème ou convertisseur (1) et le substrat (2) sont réalisés à l'aide d'une technologie "flip - chip".

3. Procédé selon la revendication 1 ou 2, **caractérisé en ce que** pour réaliser une étanchéité hermétique d'au moins une deuxième surface partielle (14) du microsystème ou convertisseur (1) qui doit être protégée des influences extérieures, on réalise entre le microsystème ou convertisseur (1) et le substrat (2) au moins un contact (51) qui entoure entièrement l'emplacement de passage (20) au moins présent dans le substrat (2).

4. Procédé selon l'une des revendications 1 à 3, **caractérisé en ce que** pour réaliser une étanchéité hermétique d'au moins une deuxième surface partielle (14) du microsystème ou convertisseur (1) qui doit être protégée des influences extérieures, un espace intermédiaire (3) entre le microsystème ou convertisseur (1) et le substrat (2) est rempli au moins en partie par un matériau de remplissage (30) d'abord liquide ou visqueux.

5. Procédé selon l'une des revendications 1 à 3 et selon la revendication 4, **caractérisé en ce que** pour réaliser une étanchéité hermétique d'au moins une deuxième surface partielle (14) du microsystème ou convertisseur (1) qui doit être protégée des influences extérieures, on réalise entre le microsystème ou convertisseur (1) et le substrat (2) au moins un contact (51) qui entoure entièrement ou partiellement l'emplacement de passage (20) au moins présent dans le substrat (2), et un espace intermédiaire (3) entre le microsystème ou convertisseur (1) et le substrat (2) est rempli au moins en partie par un matériau de remplissage (30) d'abord liquide ou visqueux.

6. Procédé selon la revendication 4 ou 5, **caractérisé en ce que** pour remplir l'espace intermédiaire (3) entre le microsystème ou convertisseur (1) et le substrat (2), on tire profit de forces capillaires.

7. Procédé selon l'une des revendications 4 à 6, **caractérisé en ce que** le matériau de remplissage (30) est durci pendant la coulée ou après la coulée.

8. Procédé selon l'une des revendications 1 à 7, **caractérisé en ce que** comme emplacement de passage (20), au moins une ouverture est créée dans le substrat (2).

9. Procédé selon la revendication 8, **caractérisé en ce que** pour maintenir la première surface partielle (13) éloignée des influences extérieures indésirables l'ouverture (20) au moins présente et recouverte par un recouvrement sélectif (24).

10. Dispositif apte à être fabriqué avec le procédé selon l'une des revendications 1 à 9, comportant un microsystème ou convertisseur (1) installé sur un substrat (2),
au moins un contact (5.1-5.3, 50, 51) se trouvant entre le microsystème ou convertisseur (1) et le substrat (2),
le microsystème ou le convertisseur (1) contenant au moins une première surface partielle (13) qui est tournée vers le substrat (2) et située face à au moins un emplacement de passage (20) dans le substrat (2) convenant pour l'interaction de la première surface partielle (13) au moins présente avec l'environnement,
le microsystème ou le convertisseur (1) contenant au moins une deuxième surface partielle (14) qui est tournée vers le substrat (2) et qui est située face à au moins une partie du substrat (2),
**caractérisé en ce que**
la deuxième surface partielle (14) au moins présente présente des entités fonctionnelles électroniques.

11. Dispositif selon la revendication 10, **caractérisé en ce qu'**au moins un contact électriquement conducteur (50) est situé entre le microsystème ou convertisseur (1) et le substrat (2).

12. Dispositif selon la revendication 10 ou 11, **caractérisé en ce qu'**entre le microsystème ou convertisseur (1) et le substrat (2) se trouve au moins un contact d'étanchéité (51) qui entoure entièrement l'emplacement de passage (20) au moins présent dans le substrat (2).

13. Dispositif selon l'une des revendications 10 à 12, **caractérisé en ce qu'**un espace intermédiaire (3) qui est rempli au moins en partie par un matériau de remplissage (30) est situé entre le microsystème ou convertisseur (1) et le substrat (2).

14. Dispositif selon la revendication 12 et 13, **caractérisé en ce qu'**entre le microsystème ou convertisseur (1) et le substrat (2) est situé au moins un contact (50) qui entoure entièrement ou partiellement l'emplacement de passage (20) au moins présent dans le substrat (2), et **en ce qu'**entre le microsystème ou convertisseur (1) et le substrat (2) est situé un espace intermédiaire (3) qui est rempli au moins partiellement par un matériau de remplissage (30).

15. Dispositif selon l'une des revendications 10 à 14, **caractérisé en ce que** l'emplacement de passage (20) est configuré comme au moins une ouverture dans le substrat.

16. Dispositif selon la revendication 15, **caractérisé par** au moins un recouvrement sélectif (24) au moyen duquel des influences extérieures indésirables peuvent être maintenues à distance de la première surface partielle (13), le recouvrement sélectif au moins présent étant situé sur, dans et/ou en dessous de l'ouverture (20) au moins présente.

17. Dispositif selon la revendication 16, **caractérisé en ce que** le recouvrement sélectif (24) est constitué d'un métal poreux ou d'une membrane partiellement perméable.

18. Dispositif selon l'une des revendications 10 à 17, **caractérisé en ce que** le microsystème où le convertisseur (1) est configuré comme puce de semi-conducteur.

19. Dispositif selon l'une des revendications 10 à 18, **caractérisé en ce que** le substrat (2) est constitué de céramique, de résine époxy, de FR4, de polyimide ou d'une autre matière synthétique.

20. Dispositif selon l'une des revendications 10 à 19, **caractérisé en ce que** les contacts (50, 51) sont constitués d'un matériau de soudure tendre ou d'un adhésif.

21. Utilisation du dispositif selon l'une des revendications 10 à 20 pour des capteurs de passage, des capteurs de viscosité, des capteurs d'humidité, des capteurs de force, des capteurs de pression, des capteurs de rayonnement électromagnétique, des capteurs de rayonnement de particules ou des capteurs chimiques.
